# EUROPEAN PATENT APPLICATION

(11) **EP 4 435 114 A1**
(43) Date of publication of application: **25.09.2024**
(21) Application number: 23163926.1
(22) Date of filing: 24.03.2023
(51) Int. Cl.: C12Q 1/6806

(54) **SYNTHEIS OF HIGHLY MULTIPLEXED OLIGO-BARCODED FLUORESCENT BEADS FOR SINGLE CELL SEQ APPLICATIONS**

(71) Applicant: Miltenyi Biotec B.V. & Co. KG, 51429 Bergisch Gladbach (DE)
(72) Inventor: MILTENYI, Stefan, 51429 Bergisch Gladbach (DE); BOSIO, Andreas, 51429 Bergisch Gladbach (DE); HARDT, Olaf, 51429 Bergisch Gladbach (DE); KERBS, Antonina, 51429 Bergisch Gladbach (DE); WAHL, Matthias, 51429 Bergisch Gladbach (DE); YUSHCHENKO, Dmytro, 51429 Bergisch Gladbach (DE)
(74) Representative: Kisters, Michael Marcus

(57) **Abstract**

The invention is directed to barcoded beads with general formula X-polynucleotide-SO, wherein X is a solid particle comprising one or more fluorescent dyes giving the solid particle a detectable color and SO is a template switching oligonucleotide comprising 3 to 30 ribonucleotides and wherein the polynucleodide comprises
- PCRhandle1: oligonucleotide comprising 4 to 40 nucleotides coding as PCR starting sequence
- C: oligonucleotide comprising 1 to 8 nucleotides coding for the detectable color of the solid particle
- UMI (unique molecular identifier) oligonucleotide comprising 5 to 15 nucleotides
- Xmer1: (bead specific barcode, part1): oligonucleotide comprising 5 to 20 nucleotides
- Xmer2: (bead specific barcode, part2): oligonucleotide comprising 5 to 20 nucleotides
**characterized in that** Xmer1 and Xmer2 are selected to provide combined at least 10e6 different sequences.

Further, the invention is directed to a method of manufacturing of such beads and their use

## Description

### BACKGROUND

The invention relates to nucleic acid probes, methods for their preparation and methods of use of the probes for single cell sequencing applications.

Rapid development of high-throughput single cell analysis techniques in combination with next generation sequencing lead recently to explosion of interest in obtaining high resolution views of single cell heterogeneity. Some of the approaches allow simultaneous measurements of gene-expression levels and cell surface epitopes in single cells providing the information about new and rare cell types, giving insights into cellular development mechanisms and protein-RNA correlation as well as cell response to therapeutic treatment.

Many of the known technologies are droplet-based platforms that utilize microfluidic systems for emulsion generation. Each water-in-oil droplet ideally encapsulates a single cell together with a bead with an oligonucleotide. After the cell lysis that takes place in the droplet, the released RNA molecules are captured by bead-oligonucleotides. Single cell analysis techniques based on beads are for example disclosed in EP3567116A1, EP3862435A1, EP2954104B1, EP3134536B1, and EP2414548B1.

The oligonucleotide on such beads typically has multiple barcodes (polynucleotide stretches that allow the identification of specific features). Such a barcode might be specific for a cell (cell specific barcode, therefore the barcode of each oligo on a particular bead has to be identical) or a molecule within a cell (unique molecular identifier). Additional feature barcodes like a color-specific barcode identifying the color of the oligo bead may be added.

The oligonucleotide on a bead additionally has features like PCR- and/ or sequencing primers, and polynucleotides required for additional biochemical reactions.

Known methods to prepare such oligo-beads include an on-bead oligo synthesis or a coupling of pre-synthesized oligo to the beads using different coupling strategies. Both methods require oligonucleotide synthesis, which is usually performed on a solid support using a DNA synthesizer. Synthesis of long oligos is low yielding (the yield *per* chain elongation step is mostly around 99%) and time consuming. Moreover, it can become very expensive if to take into account that for a sufficient single cell transcriptome and proteome analysis more than one million of different barcoded oligo-beads are required. This is especially crucial for single cell m-RNA sequencing.

Object of the invention was to manufacture oligo-beads capable of providing millions of different barcodes with an economically feasible process and using such oligo-beads in single-cell m-RNA sequencing procedures.

### SUMMARY

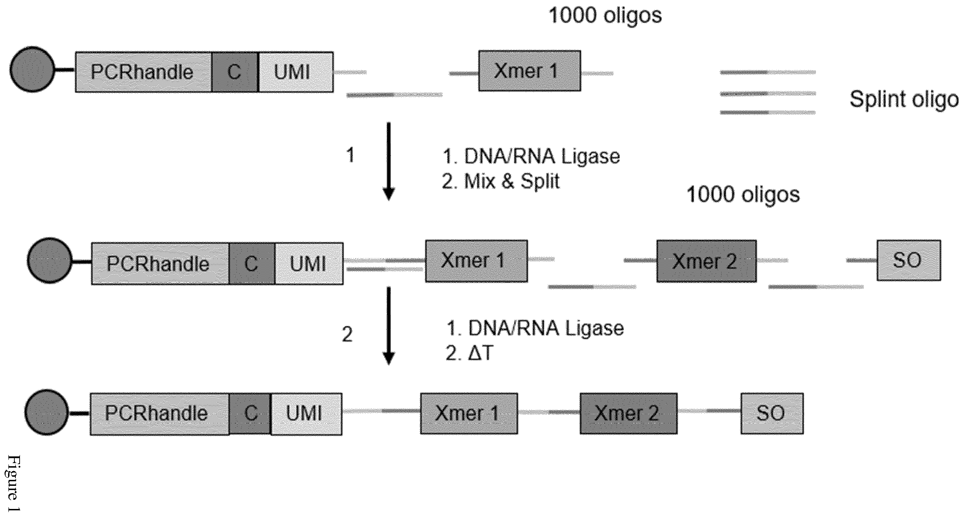

To solve this problem, a new approach for the synthesis of oligo-beads is proposed. The attachment of oligos bearing a PCR primer, UMI and the color code will be performed using a standard chemistry. The coupling of the first part of a barcoded oligonucleotide will be realized by enzymatic ligation with a phosphorylated Xmer1 (Figure 1). For a sufficient ligation, hybridization of a short sequence oligonucleotide (splint) complementary to six nucleobases of an oligo coupled to the bead as well as to the six nucleobases of the Xmer1 is first performed, followed by ligation reaction. Prepared oligo-bead construct can be ligated in the same manner with the next barcode part of a different sequence (Xmer2). In this way thousands of Xmer1 could be coupled with thousands of Xmer2 resulting in more than a million of different barcoded oligo-bead constructs. As a last step the switching oligo can be ligated to the barcoded oligo-bead construct forming a bead-oligo sequence capable to capture an RNA molecule from the cell.

Accordingly, object of the invention are barcoded beads with the general formula X-polynucleotide-SO, wherein X is a solid particle comprising one or more fluorescent dyes giving the solid particle a detectable color and SO is a template switching oligonucleotide comprising 3 to 30 ribonucleotides and wherein the polynucleodide comprises
- PCRhandle1: oligonucleotide comprising 4 to 40 nucleotides coding as PCR starting sequence
- C: oligonucleotide comprising 1 to 8 nucleotides coding for the detectable color of the solid particle
- UMI (unique molecular identifier) oligonucleotide comprising 5 to 15 nucleotides
- Xmer1: (bead specific barcode, part1): oligonucleotide comprising 5 to 20 nucleotides
- Xmer2: (bead specific barcode, part2): oligonucleotide comprising 5 to 20 nucleotides characterized in that Xmer1 and Xmer2 are selected to provide combined at least 10e6 different sequences.

The UMI (unique molecular identifier) oligonucleotide can be used to provide error correction during sequencing.

### BRIEF DESCRIPTION OF THE DRAWINGS

Figure 1 shows the preparation of oligo-bead constructs by ligation on beads using a three splint approach resulting at least one million of different oligo-beads.
Figure 2 shows the preparation of oligo-bead constructs by ligation on beads using a two splint approach. The SO oligonucleotide is first ligated to Xmer2 using the universal splint and the resulting oligo is subsequently ligated to Xmer1 resulting at least one million of different oligo-beads.
Figure 3 shows the preparation of oligo-bead constructs by ligation on beads using a one splint approach where Xmer1 is first chemically coupled to the beads and then a phosphorylated oligonucleotide containing Xmer2 and switching oligo is ligated to the oligo-beads. For one splint approach one splint is used.
Figure 4 shows the preparation of oligo-bead constructs by ligation on beads using a two bases overhangs two splint approach where Xmer1 is first chemically coupled to the beads and then a phosphorylated oligonucleotide containing Xmer2, UMI, C and switching oligo is ligated to the oligo-beads.
Figure 5 shows the preparation of oligo-bead constructs by ligation on beads using a no splint approach where Xmer1 is first chemically coupled to the beads. The phosphorylated Xmer2-containing oligonucleotide is first adenylated and subsequently ligated to Xmer1 oligo-beads without using a splint.

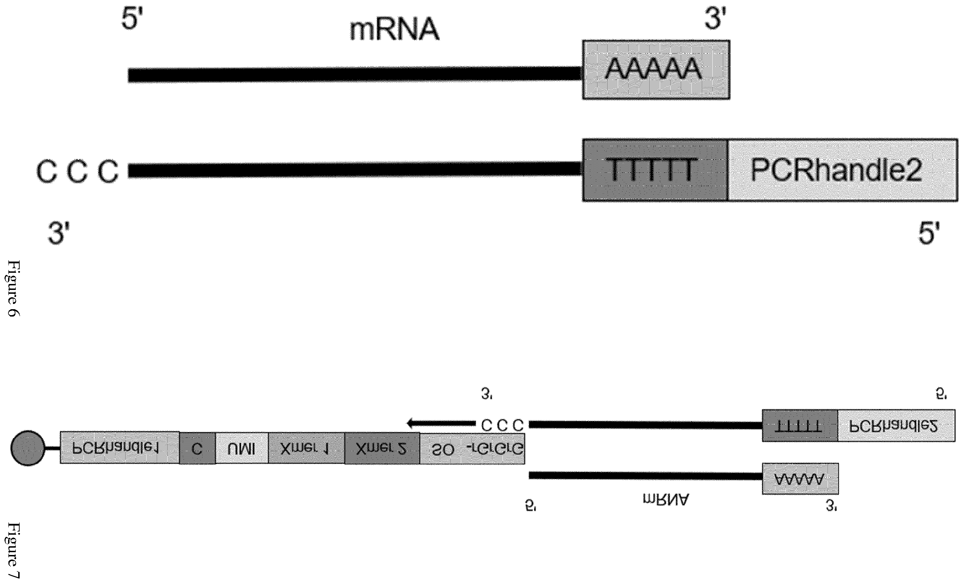

Figure 6 shows the capturing of mRNA by hybridization a complementary strand to the m-RNA wherein the complementary strand is provided at its 3' end with a poly-T sequence matching the poly A sequence of the m-RNA as well as a PCR handle sequence and at its 5' end a poly-C sequence.
Figure 7 shows the binding of switching oligo sequence (SO) to the poly C sequence of the oligonucleotide complementary to mRNA sequence.

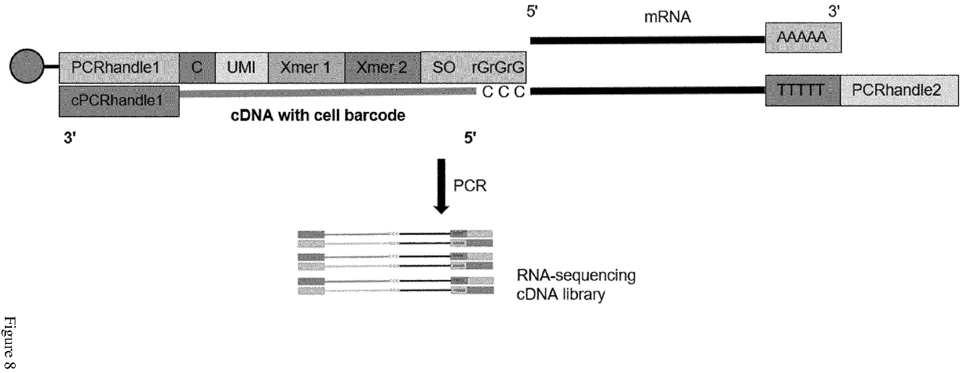

Figure 8 shows the addition of nucleotides to the complementary strand at its 5' end until the complementary strand matches the oligonucleotide.

### DETAILED DESCRIPTION

### Barcoded beads

The term "barcode" is applied to C, UMI, Xmer1 and Xmer2 because it allows the identification of a single target by its unique sequence.

In general, the barcoded beads have the composition X-polynucleotide-SO, wherein "polynucleotide" comprises the units X, PCRhandel1, Xmer1, Xmer2, UMI, and C (as already disclosed). These units may be bound to each other directly or via the same or a different spacer oligonucleotide comprising 1 to 30 nucleotides.

The sequence of the units X, PCRhandel1, Xmer1, Xmer2, UMI, and C is not of importance for the use of the barcoded beads. However, the barcoded beads have preferable a composition according to one or more of the following general formulas (I-VI):
X-PCRhandle-C-UMI-Xmer1-Xmer2-SO (I)
X-PCRhandle-UMI-C-Xmer1-Xmer2-SO (II)
X-PCRhandle-UMI-Xmer1-Xmer2-C-SO (III)
X-PCRhandle-C-Xmer1-Xmer2-UMI-SO (IV)
X-PCRhandle-Xmer1-Xmer2-C-UMI-SO (V)
X-PCRhandel-Xmer1-Xmer2-UMI-C-SO (VI)

The PCR handle may comprise 4 to 30 nucleotides and serves as a primer binding region for subsequent amplification reactions.

The PCR handle may contain an oligonucleotide comprising 1 to 10 nucleotides (such as restriction enzyme site(s)) allowing for additional biochemical reactions other than amplification reactions.

The color specific barcode C may comprise 1 to 8 nucleotides and allows the identification of a cell or a cell type.

The beads specific barcode containing Xmer1 and Xmer2 may comprise 10-40 nucleotides and serves as a cell specific barcode allowing the assignment of the sequencing data to the origin cell. The first barcode sequence Xmer1 together with the second barcode sequence Xmer2 can provide 10000 to 500×10e6 different bead specific barcodes.

The unique molecular identifier (UMI) may comprise 5 to 15 nucleotides and serves as an identifier for each single nucleic acid molecule in the target cell.

The switching oligonucleotide may comprise 3 to 30 DNA and/or RNA nucleotides and serves as a binding region for untemplated cytosine nucleotides added by the reverse transcriptase during reverse transcription. The switching oligo adds the sequences of C, Xmer1, Xmer2, UMI and PCR oligonucleotides to full length cDNA that is used in cDNA amplification reactions.

The oligonucleotides PCR, C, UMI, Xmer1, Xmer2 and SO may be prepared by utilizing any of the commercially available DNA synthesizers.

In a first variant of the invention, at least one of the PCRhandlel, C, UMI, Xmer1, Xmer2 and SO is provided with an adenosine monophosphate group in the 5' position.

In a second variant of the invention, at least one of the PCRhandlel, C, UMI, Xmer1, Xmer2 and SO is provided with a phosphate group in the 5' position.

The PCRhandle1 may be covalently bound to an oligonucleotide sequence containing a restriction endonuclease restriction site.

In a third variant of the invention the template switching oligonucleotide SO comprises the sequence rGrGrG.

The oligonucleotide SO may contain 3 to 30 ribonucleotides in the 3' position.

### Method of manufacture

Further object of the invention is a method of manufacture the barcoded beads characterized in that the polynucleotide is obtained by ligating Xmer1 and/or Xmer2 via a short sequence oligonucleotide (splint) complementary to a sequence of 0 to 30 nucleotides.

In short, the method of manufacture according to the invention may comprise the features:
- Splitting barcode into two parts permits high diversity that can be achieved with descent number of ligations
- combining different parts of oligo to achieve efficient ligation without having to synthesize extra-long oligos
- high density of oligos on beads that ensures efficient capturing and barcoding of mRNA molecules.

The coupling of an oligonucleotide to the bead can be performed using chemical and/or enzymatic coupling reactions.

In a first embodiment, the coupling of the first barcode oligonucleotide Xmer1 to oligo-beads with one of the general formulas (Ia to Va):
X-PCRhandle1-C-UMI (Ia)
X-PCRhandle1-UMI-C (IIa)
X-PCRhandle1-UMI (IIIa)
X-PCRhandle1-C (IVa)
X-PCRhandle1 (Va)
may be performed using enzymatic ligation reactions providing oligo-beads with one of the general formulas (Ib to Vlb):
X-PCRhandle1-C-UMI-Xmer1 (Ib)
X-PCRhandle1-UMI-C-Xmer1 (IIb)
X-PCRhandle1-UMI-Xmer1 (IIIb)
X-PCRhandle1-C-Xmer1 (IVb)
X-PCRhandle1-Xmer1 (Vb)
X-PCRhandel1-Xmer1 (VIb).

The oligonucleotides Ib to VIb may be coupled directly to the beads using standard chemical coupling procedures.

The coupling of the second barcode oligonucleotide Xmer2 to oligo-beads Ib to VIb may be performed using enzymatic ligation reactions providing oligonucleotides with one of the general formulas (Ic to VIc):
X-PCRhandle1-C-UMI-Xmer1-Xmer2 (Ic)
X-PCRhandle1-UMI-C-Xmer1-Xmer2 (IIc)
X-PCRhandle1-UMI-Xmer1-Xmer2 (IIIc)
X-PCRhandle1-C-Xmer1-Xmer2 (IVc)
X-PCRhandle1-Xmer1-Xmer2 (Vc)
X-PCRhandel1-Xmer1-Xmer2 (VIc)

The coupling of the SO oligonucleotide to oligo-beads Ic and IIc may be performed using enzymatic ligation reactions providing oligonucleotides with one of the general formulas I to II. Further, the coupling of SO oligonucleotide, bound chemically or enzymatically to either C or UMI or C and UMI, with oligo-beads IIIc or IVc or Vc or VIc correspondingly may be performed using enzymatic ligation reactions providing oligonucleotides with one of the general formulas (III to VI).

In another embodiment of the method oligo-beads Ib to VIb may be coupled with Xmer2 oligonucleotides which are chemically or enzymatically bound to either C or UMI or C and UMI, each coupled with SO, providing oligonucleotides with one of the general formulas I to VI.

In this way, thousands of Xmer1 could be coupled with thousands of Xmer2 resulting a million of different barcoded oligo-bead constructs.

For the enzymatic ligation reaction, preferably hybridization of a short sequence oligonucleotide (splint) complementary to a sequence of 0 to 30 nucleobases may be performed. The hybridization of the splint is preferably performed before the enzymatic ligation reactions.

The hybridized splint may be removed by heating or replacement using a complementary oligonucleotide.

Additionally, the entire oligonucleotide sequences (a to f), chemically modified to have an amino or a thiol group, may be directly coupled to the beads using standard chemical reactions such as amide coupling or thiol Michael addition reactions.
PCRhandle1-C-UMI-Xmer1-Xmer2-SO (a)
PCRhandle1-UMI-C-Xmer1-Xmer2-SO (b)
PCRhandle1-UMI-Xmer1-Xmer2-C-SO (c)
PCRhandle1-C-Xmer1-Xmer2-UMI-SO (d)
PCRhandle1-Xmer1-Xmer2-C-UMI-SO (e)
PCRhandel1-Xmer1-Xmer2-UMI-C-SO (f)

Further, the sequences (a to f) may be synthesized directly on the beads using standard oligonucleotide synthesis reactions.

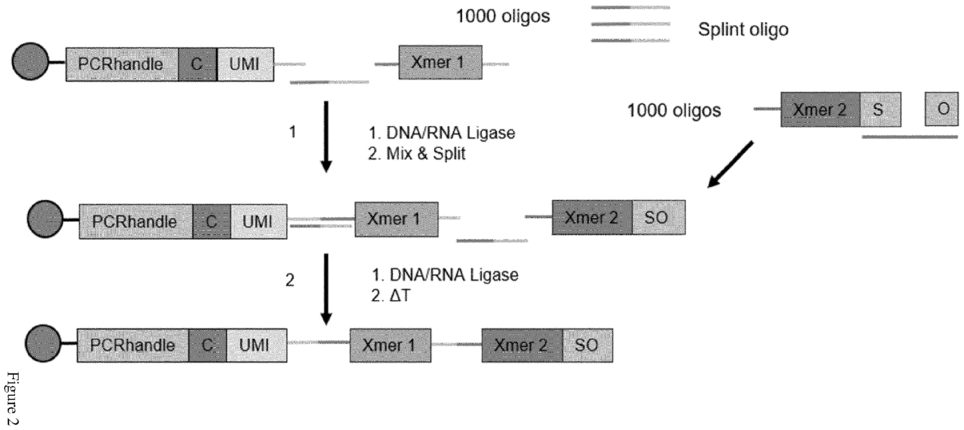

Alternatively, two, one and no splint ligation approaches can be used to prepare one million of different barcoded oligonucleotides on the bead. In the two-splint approach, the switching oligo is first ligated to Xmer2 using the universal splint and the resulting oligo is subsequently ligated to Xmer1 which was previously enzymatically attached to oligo-beads (Figure 2).

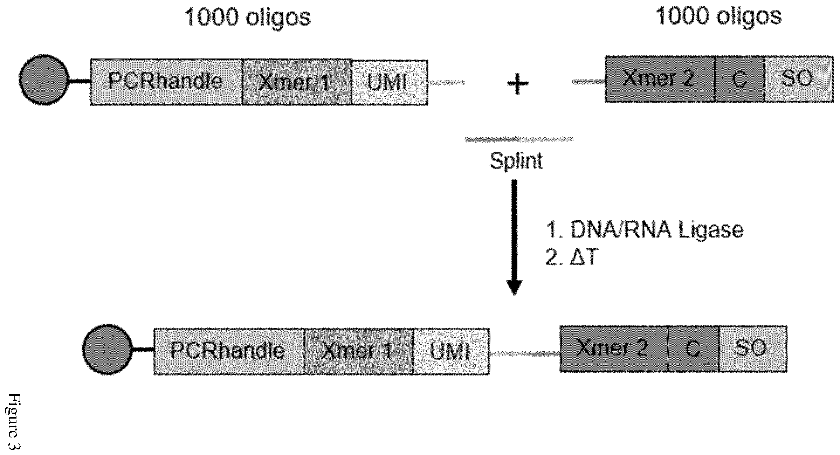

For the one and no splint approaches Xmer1 is coupled to the beads first and then a phosphorylated oligonucleotide containing Xmer2 and switching oligo is ligated to the oligo-beads. For one splint approach one splint is used (Figure 3).

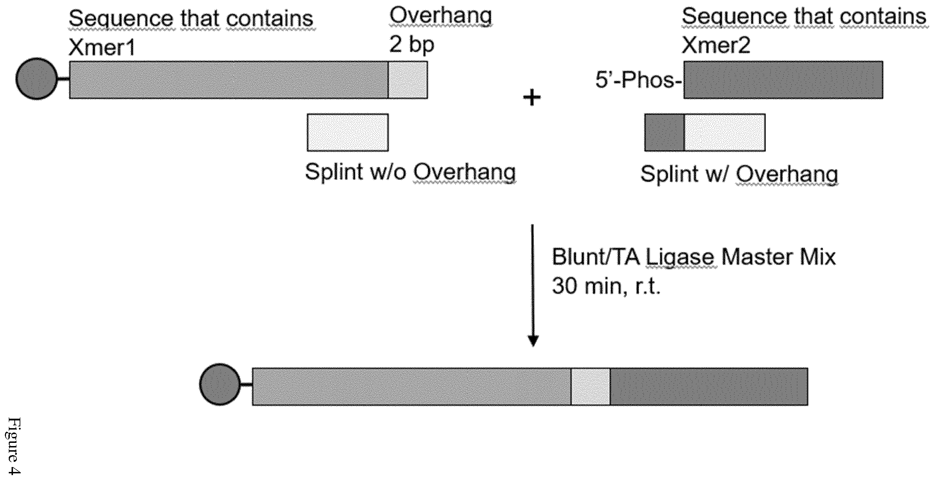

In case of two bases overhang two splint approach two splints are used. One splint hybridizes to the Xmer1 sequence except two bases at the 3'-position, which are hybridized to the second splint that is complementary to the Xmer2 oligonucleotide (Figure 4).

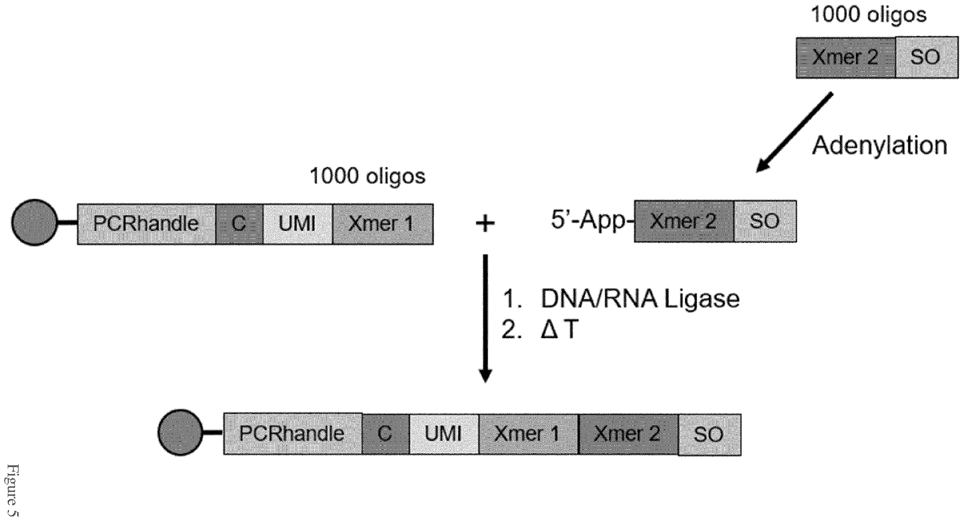

In case of no splint approach the phosphorylated Xmer2-containing oligonucleotide is first adenylated and subsequently ligated to oligo-beads without using a splint (Figure 5).

In a variant of the method, the short sequence oligonucleotide (splint) is removed from the polynucleotide by heating.

Further, it is possible that the polynucleotide is obtained by ligating PCRhandlel, C, UMI and SO using an enzymatic ligation reaction.

### Solid Particle X

The size of the solid particle X may be between 1 and 20 µm.

Solid particles used in this invention may be manufactured using any kind of material which is compatible with the employed system. Such a material could be for example silicate, polystyrene, polydextran, polyethyleneglycol, polyethyleneimine or other optionally chemically modified with reactive groups to bind dyes and/or oligonucleotides. Suitable reactive groups are for example carboxylic, amino or maleimide groups.

Solid particles may be prepared by incorporation of dyes into pre-formed polymeric structures by swelling them in organic solvent mixtures containing dyes (US 6514295 B1, US 7507588 B2).

Another method employs the hydrophobic interactions of dyes with the polymeric bead material caused by shifting of phase equilibria upon addition of water to the system.

Further, solid particles can be prepared by polymerization of monomer mixtures containing fluorescent monomers (JACS 2004, 126, 21, 6562-6563).

The concentration and the difference in emission maxima of the dyes are selected in a way that the discrimination of at least 2 different solid particles is possible.

Dyes including fluorescent proteins, polymers, small molecules, clusters of fluorescent proteins or small organic molecules as well as metal-organic complexes and fluorescent nanoparticles may be used as fluorescent entities.

Solid particles may comprise multiple subunits linked via magnetic units, electrostatic interaction or covalent and/or non-covalent linkage. These subunits may be released from each other upon droplet formation for example by photo-/chemical and/or enzymatic cleavage.

### Use of the barcoded beads for sequencing mRNA

Further object of the invention is the use of the barcoded beads according to the invention in a method for sequencing mRNA from a plurality of cells characterized in
- Isolating cells into compartments
- Providing the isolated cells with first oligonucleotide comprising a poly-T sequence and a PCR starting sequence (PCRhandle2) comprising 4 to 40 nucleotides and binding the m-RNA from the isolated cell via their poly-A sequences to the first oligomers enzymatically completing the strand originating from the first oligonucleotide with complementary nucleotides using the bond m-RNA strand as template thereby creating c-DNA strands containing a poly-C sequence overhangs
- Providing one or more barcoded beads wherein the barcoded beads hybridize via the SO sequence to the poly-C sequence of the c-DNA
- completing the strand of the c-DNA originating from the first oligonucleotide with complementary nucleotides using the polynucleotide of the oligo-bead as template
- amplifying and sequencing the complementary DNA oligonucleotide

### EXAMPLES

Example 1: Preparation of oligo-bead constructs (III) by ligation on beads using a one splint approach where oligo bearing Xmer1 is first chemically coupled to the beads and then a phosphorylated oligonucleotide containing Xmer2 and switching oligo is ligated to the oligo-beads.

First, the oligo-bead constructs with the general formula (IIIb) were prepared using the thiol-maleimide coupling reaction. These were then ligated with the Xmer2-SO oligonucleotide to obtain the oligo-beads (III). For the first step, the thiolated oligonucleotide (Metabion) was reduced using the TCEP reducing reagent. Therefore, 60 nmol thiolated oligo was incubated in 120 µL MilliQ water with 600 nmol TCEP at room temperature for 2 h. The thiol protective group (alkylthiol) and the excess of the reducing reagent were removed by washing the reaction mixture using the 10 kDa Amicon filters (Th. Geyer) as well as NAP^{™}-5 column (Cytiva illustra^{™}) and PBS/0.03% pluronic (F-68) buffer. The purified thiolated oligonucleotide 5 µM was added immediately to 1×10⁷ maleimide functionalized polystyrene beads in PBS/0.03% pluronic (F-68) and the mixture with the final volume of 200 µL was incubated at RT for 12 h. The oligo-bead construct with the general formula (IIIb) was washed three times with PBS/0.03% pluronic (F-68) to remove the unreacted oligo.

In the next step the oligonucleotide containing Xmer2 and SO sequences was ligated to the oligo-beads (IIIb) to obtain the oligo-bead constructs with the general structure (III). Therefore, 1×10⁶ previously prepared oligo-beads (IIIb) were resuspended in 150 µL PBS/0.03% pluronic (F-68) buffer and pre-hybridized at 85 °C for 5 min with 0,2 nmol of the phosphorylated Xmer2SO oligonucleotide using 0,2 nmol splint oligonucleotide. After bringing the reaction mixture to an ambient temperature the resulted oligo-beads were centrifuged in order to remove the hybridization buffer and resuspended in the ligation buffer (Quick ligation^{™} kit - NEB). Afterwards the T4 ligase (4U) was added and the reaction mixture was incubated for 30 min at room temperature. In order to remove the splint oligonucleotide the anti-splint oligonucleotide (0,3 nmol, sequence complementary to the splint oligonucleotide sequence) was added and the reaction mixture was heated to 70 °C for 20 min. The resulting oligo-beads (III) were washed three times with PBS/0.03% pluronic (F-68) buffer and stored at -70 °C until further use.

Example 2: Preparation of oligo-bead constructs by ligation on beads using a two bases overhangs two splint approach where Xmer1 is first chemically coupled to the beads and then a phosphorylated oligonucleotide containing Xmer2, UMI, C and switching oligo is ligated to the oligo-beads.

First, the oligo-bead constructs with the general formula (VIb) were prepared using the thiol-maleimide coupling reaction. These were then ligated with the Xmer2-UMI-C-SO oligonucleotide to obtain the final oligo-beads (VI). For the first step, thiolated oligonucleotide (Metabion) was reduced using the THPP reducing reagent. Therefore, 142 nmol thiolated oligonucleotide was incubated in 1,70 mL PBS buffer with 1420 nmol THPP at room temperature for 1 h. The thiol protective group (alkylthiol) and the excess of the reducing reagent were removed by washing the reaction mixture using the 10 kDa Amicon filters (Th. Geyer) as well as NAP^{™}-5 column (Cytiva illustra^{™}) and PBS/0.03% pluronic (F-68) buffer. The purified thiolated oligonucleotide (130 nmol, 250 µM in PBS/0.03% pluronic (F-68)) was added immediately to 26×10⁶ maleimide functionalized polystyrene beads and the mixture was incubated at RT for 2 h. The oligo-bead constructs with the general formula (VIb) was washed three times with PBS/0.03% pluronic (F-68) to remove the unreacted oligo.

In the next step the oligonucleotide containing Xmer2, UMI, C and SO sequences was ligated to the oligo-beads (VIb) to obtain the oligo-bead constructs with the general structure (VI). Therefore, 6×10⁶ previously prepared oligo-beads (VIb) were ligated with 3 nmol Xmer2-UMI-C-SO oligonucleotide using the Xmer1 and Xmer2 splints (3 nmol each) as well as 90 µL Blunt/TA ligase MasterMix in the final reaction volume of 180 µL. The reaction mixture was incubated at an ambient temperature for 30 min. The resulting oligo-beads were washed three times with PBS/0.03% pluronic (F-68) buffer and stored at -70 °C until further use.

Example 3: Evaluation of the functionality of the oligo-beads by conducting a reverse transcription reaction with a template switching using cells as an input.

For this, 16100 cells and the same amount of oligo-beads were incubated in a reaction mixture containing a restriction enzyme (0.4 U/µl) to mediate the enzymatic release of the bead bound oligo, an oligo(dT) primer (2µM) containing a second primer binding site and a modified MMLV reverse transcriptase (10 U/µl). The mixture was incubated for 45 minutes at 37°C.

Subsequently, the c-DNA was amplified using the Q5^{®} High-Fidelity 2X Master Mix (Cat. No. M0515, New England Biolabs, Ipswich, MA, USA) and amplified for 14 cycles using:

A first primer specific for the universal sequence, introduced to the c-DNA by template switching using the template switching oligo, delivered by the bead and

A second primer specific for the second primer binding site introduced via the oligo(dT) primer of the reverse transcription reaction.

The final yield, which serves as a proxy for the efficiency of the reverse transcription reaction, was quantified by measuring the amount of c-DNA using the Thermo Fisher Qubit dsDNA HS-Assay-Kit (Cat. No. Q32851, Thermo Fisher, Waltham, MA, USA).

## Claims

1. Barcoded beads with the general formula X-polynucleotide-SO, wherein X is a solid particle comprising one or more fluorescent dyes giving the solid particle a detectable color and SO is a template switching oligonucleotide comprising 3 to 30 ribonucleotides and wherein the polynucleodide comprises
- PCRhandle1: oligonucleotide comprising 4 to 40 nucleotides coding as PCR starting sequence
- C: oligonucleotide comprising 1 to 8 nucleotides coding for the detectable color of the solid particle
- UMI (unique molecular identifier) oligonucleotide comprising 5 to 15 nucleotides
- Xmer1: (bead specific barcode, part1): oligonucleotide comprising 5 to 20 nucleotides
- Xmer2: (bead specific barcode, part2): oligonucleotide comprising 5 to 20 nucleotides
**characterized in that** Xmer1 and Xmer2 are selected to provide combined at least 10e6 different sequences.

2. Barcoded beads according to claim 1 **characterized in that** the barcoded beads have the sequence compositions according to one of the general formulas (I) to (VI)
X-PCRhandle1-C-UMI-Xmer1-Xmer2-SO (I)
X-PCRhandle1-UMI-C-Xmer1-Xmer2-SO (II)
X-PCRhandle1-UMI-Xmer1-Xmer2-C-SO (III)
X-PCRhandle1-C-Xmer1-Xmer2-UMI-SO (IV)
X-PCRhandle1-Xmer1-Xmer2-C-UMI-SO (V)
X-PCRhandel1-Xmer1-Xmer2-UMI-C-SO (VI)

3. Barcoded beads according to claim 1 or 2 **characterized in that** the solid particles comprise silicate, polystyrene, polydextran, polyethylene glycol or polyethyleneimine.

4. Barcoded beads according to at least one of the claims 1 to 3**characterized in that** the solid particles have a mean diameter of 1 and 20 µm.

5. Barcoded beads according to at least one of the claims 1 to 4 **characterized in that** the template switching oligonucleotide SO comprises the sequence rGrGrG.

6. Barcoded beads according to at least one of the claims 1 to 5 **characterized in that** at least one of the PCRhandlel, C, UMI, Xmer1, Xmer2 and SO is provided with an adenosine monophosphate group in the 5' position.

7. Barcoded beads according to at least one of the claims 1 to 5 **characterized in that** at least one of the PCRhandlel, C, UMI, Xmer1, Xmer2 and SO is provided with a phosphate group in the 5' position.

8. Barcoded beads according to at least one of the claims 1 to 7 **characterized in that** PCRhandle1 is covalently bound to an oligonucleotide sequence containing a restriction endonuclease restriction site.

9. Method of manufacture the barcoded beads according to any of the claims 1 to 8 **characterized in that** the polynucleotide is obtained by ligating Xmer1 and/or Xmer2 via a short sequence oligonucleotide (splint) complementary to a sequence of 0 to 30 nucleotides.

10. Method according to claim 9, **characterized in that** the short sequence oligonucleotide (splint) is removed from the polynucleotide by heating

11. Method according to claim 9 or 10, **characterized in that** the polynucleotide is obtained by ligating PCRhandlel, C, UMI and SO using an enzymatic ligation reaction.

12. Use of the barcoded beads according to any of the claims 1 to 8 in a method for sequencing mRNA from a plurality of cells **characterized in**
a) Isolating cells into compartments
b) Providing the isolated cells with first oligonucleotide comprising a poly-T sequence and a PCR starting sequence (PCRhandle2) comprising 4 to 40 nucleotides and binding the m-RNA from the isolated cell via their poly-A sequences to the first oligomers
c) enzymatically completing the strand originating from the first oligonucleotide with complementary nucleotides using the bond m-RNA strand as template thereby creating c-DNA strands containing a poly-C sequence overhangs
d) Providing one or more barcoded beads wherein the barcoded beads hybridize via the SO sequence to the poly-C sequence of the c-DNA
e) completing the strand of the c-DNA originating from the first oligonucleotide with complementary nucleotides using the polynucleotide of the oligo-bead as template
amplifying and sequencing the complementary DNA oligonucleotide
